# EUROPEAN PATENT APPLICATION

(11) **EP 3 831 963 A1**
(43) Date of publication of application: **09.06.2021**
(21) Application number: 19845314.4
(22) Date of filing: 28.06.2019
(51) Int. Cl.: C12Q 1/6886

(54) **BREAST CANCER EARLY DIAGNOSIS AND POST-THERAPY MONITORING METHOD USING LIQUID BIOPSY MULTIPLE CANCER GENE BIOMARKERS**

(30) Priority: 02.08.2018 KR 20180090451
(71) Applicant: Exogen Pte. Ltd, International Plaza 079903 (SG)
(72) Inventor: JANG, Ji Young, Seoul 06608 (KR)
(74) Representative: Dehns
(86) International application number: PCT/KR2019/007906
(87) International publication number: WO 2020/027446

(57) **Abstract**

The present invention relates to a method for early diagnosis and post-treatment monitoring for each molecular subtype of breast cancer using liquid biopsy multiple gene biomarkers, and more particularly, to a non-invasive method for early diagnosis and post-treatment monitoring for breast cancer and each molecular subtype of breast cancer using exosomes. That is, the present inventors have developed a technology for analyzing mRNA inside the exosome, and determined that the accuracy and reproducibility of diagnosis can be improved through the above technology. As such, through the minimal multiple cancer gene biomarker technology applicable to the early diagnosis of breast cancer according to the each molecular subtypes, a method for early diagnosis of breast cancer according to four molecular subtypes thereof, a method of determining the degree of breast cancer progression depending on the degree of expression levels, and the like can be provided. Thus, the present invention can be widely used in the development of a method for early diagnosis of breast cancer using liquid biopsy multiple cancer gene biomarkers and a medical instrument for ex-vivo gene diagnosis for monitoring recurrence after treatment of breast cancer.

## Description

### [Technical Field]

The present invention relates to a method for early diagnosis of breast cancer and post-treatment monitoring method using multiple cancer gene biomarkers, and more particularly, to a non-invasive method for early diagnosis of breast cancer and monitoring after treatment using multiple gene expression analysis in exosomes.

### [Background Art]

Breast cancer has the highest cancer incidence rate among women in the world and is the second most common cancer among women after thyroid cancer in Korea, X-ray-based mammography with excellent microcalcification sensitivity for diagnosing breast cancer is usually used, and women with dense breasts, known as a risk factor for developing breast cancer, have a limit of remarkably reduced sensitivity to X-ray mammography, so that there are disadvantages in that additional breast ultrasonography should be performed, and due to the characteristics of ultrasonic testing, the testing time is relatively long, and the ultrasonic testing is highly dependent on a tester. Further, an invasive method of breast cancer diagnosis by breast cancer tissue biopsy has problems in that an examinee suffers from pain, there are side effects due to infection, and hospitalization and a recovery period after the test are required. Therefore, the development of an early diagnosis method for breast cancer using a liquid biopsy is very urgent, and is expected to reduce the number of tissue biopsies and overcome the low sensitivity of X-ray mammography.

Since breast cancer diagnostic methods using a liquid biopsy, which have been developed to date, use only a small number of biomarkers for diagnosis and most biomarkers show high sensitivity in only stage 4 breast cancer, there is great difficulty in early diagnosis, so that a new cancer genetic diagnosis method using body fluids has been actively developed worldwide. As a representative breast cancer genetic diagnosis technique using a liquid biopsy, there is a genetic test technique that analyzes circulating tumor DNA (ctDNA) in blood released from tumor cells as a result of death of tumor cells in blood. This technique (ctDNA gene analysis method) is a method that usually analyzes the presence or absence of the mutations of a specific gene (BRACA1/2 mutation), is a technique which is more suitable for predicting the possibility of carcinogenesis in the future than early diagnosis of cancer, and is a very effective technique for selecting a therapeutic agent tailored to an individual by predicting the therapeutic effect of an anticancer agent (HER2 target therapy). However, in the case of small tumors in the early stages, the half-life of ctDNA in blood is within 1 hour and has low stability and reproducibility of a result, the level of ctDNA varies from individual to individual and it is difficult to detect ctDNA (low detection efficiency, low purity, and low sensitivity), so that there is a need for developing a highly sensitive detection method.

Meanwhile, periodic examinations are required to confirm the presence or absence of recurrence even after breast cancer surgery, but recurrence after breast cancer surgery is divided into two types of (1) local recurrence and (2) systemic recurrence (distant metastasis). Local recurrence refers to recurrence in the surgical site, chest wall, axillary lymph glands, and the like, and systemic recurrence refers to recurrence in other organs of the body such as the lungs, bones, liver, brain, and ovaries. Since recurrences are most likely to occur within 5 years after surgery, a periodic examination should be performed at least once every 6 months for 5 years and then once a year for the rest of one's life. A representative periodic breast cancer examination includes (1) seeing a doctor (to observe whether new cancer develops in other parts of breast and the other breast), (2) breast photography, (3) a tumor marker blood test (CA15-3), (4) a liver function blood test, (5) a chest lung photograph (to observe whether there is metastasis in the lungs), (6) a systemic isotope bone scan (to observe whether there is metastasis in the bones, but is performed when abnormalities are found by a tumor marker test and a liver function test, or when there are symptoms such as bone pain and is regularly performed at an interval of 6 months on only a patient with stage 2 or higher breast cancer), (7) liver ultrasonography (performed only when it is suspected that an examination is needed), and (8) brain computed tomography (CT) (performed only when it is suspected that an examination is needed). However, as described above, the regular examination items for monitoring the recurrence of breast cancer, which are currently being performed, have disadvantages of not only being expensive, but also causing inconvenience to an examinee and requiring a lot of time. Therefore, there is an urgent need for developing a post-treatment monitoring method capable of obtaining results with high detectability and low cost, simply and in a short time without pain to an examinee.

### [Disclosure]

### [Technical Problem]

The present invention has been devised to solve the above-described problems in the related art, and an object of the present invention is to provide an early diagnosis method for breast cancer and molecular subtypes of breast cancer using mRNA in exosomes to measure the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

Further, an object of the present invention is to provide a method for monitoring recurrence after treatment of breast cancer using mRNA in exosomes to measure the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

However, the technical problems which the present invention intends to solve are not limited to the technical problems which have been mentioned above, and other technical problems which have not been mentioned will be clearly understood by a person with ordinary skill in the art to which the present invention pertains from the following description.

### [Technical Solution]

To achieve the above-described objects of the present invention, the present invention provides a method for providing information for early diagnosis of breast cancer, the method including: (a) extracting mRNA from exosomes isolated from biological samples of a normal person and a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2 and VDAC 1 genes are increased compared to the normal person as breast cancer.

Further, the present invention provides a breast cancer early diagnosis method including: (a) extracting mRNA from exosomes isolated from biological samples of a normal person and a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2 and VDAC1 genes are increased compared to the normal person as breast cancer.

As an exemplary embodiment of the present invention, the breast cancer may be selected from the group consisting of Luminal A, Luminal B, HER2 type, and Triple negative/basal-like molecular subtypes.

In addition, the present invention provides a method for providing information for early diagnosis of molecular subtype Luminal A breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2, VDAC1, and HCCR1 genes are increased compared to the mRNA levels of caveolin-1, β-catenin, and TGF-β3 genes as molecular subtype Luminal A breast cancer.

Furthermore, the present invention provides a method for early diagnosis of molecular subtype Luminal A breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and TGF-β3 genes using the extracted mRNA as a template; and (c) determining a case where the mRNA levels of the ANT2, VDAC1, and HCCR1 genes are increased compared to the mRNA levels of caveolin-1, β-catenin, and TGF-β3 genes as molecular subtype Luminal Abreast cancer.

As an exemplary embodiment of the present invention, the molecular subtype Luminal A type is estrogen receptor-positive (ER-positive), progesterone receptor-positive (PR-positive), and human epidermal growth factor receptor 2-negative (HER2-negative).

Further, as another exemplary embodiment of the present invention, the subject is a patient with breast cancer.

In addition, the present invention provides a method for providing information for early diagnosis of molecular subtype Luminal B breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2, VDAC1, and TGF-β3 genes are increased compared to the mRNA levels of caveolin-1, and β-catenin genes as molecular subtype Luminal B breast cancer.

Furthermore, the present invention provides a method for early diagnosis of molecular subtype Luminal B breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2, VDAC1, and TGF-β3 genes are increased compared to the mRNA levels of HCCR1, caveolin-1, and β-catenin genes as molecular subtype Luminal B breast cancer.

As an exemplary embodiment of the present invention, the molecular subtype Luminal B type is ER-positive, PR-positive, and HER2-positive.

Further, the present invention provides a method for providing information for early diagnosis of molecular subtype HER2 type breast cancer, the method including:
(a) extracting mRNA from exosomes isolated from a biological sample of a subject;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2, VDAC1, and TGF-β3 genes are increased compared to the mRNA levels of HCCR1, caveolin-1, and β-catenin genes as molecular subtype HER2 type breast cancer.

In addition, the present invention provides a method for early diagnosis of molecular subtype HER2 type breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2, VDAC1, and TGF-β3 genes are increased compared to the mRNA levels of HCCR1, caveolin-1, and β-catenin genes as molecular subtype HER2 type breast cancer.

As an exemplary embodiment of the present invention, the molecular subtype HER2 type is ER-negative, PR-negative, and HER2-positive.

Furthermore, the present invention provides a method for providing information for early diagnosis of molecular subtype Triple negative/basal-like breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA level of the caveolin-1 gene is increased compared to the mRNA levels of ANT2, VDAC1, HCCR1, β-catenin, and TGF-β3 genes as molecular subtype Triple negative/basal-like breast cancer.

Further, the present invention provides a method for early diagnosis of molecular subtype Triple negative/basal-like breast cancer, the method including: (a) extracting mRNA from exosomes isolated from a biological sample of a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA level of the caveolin-1 gene is increased compared to the mRNA levels of ANT2, VDAC1, HCCR1, β-catenin, and TGF-β3 genes as molecular subtype Triple negative/basal-like breast cancer.

As an exemplary embodiment of the present invention, the molecular subtype Triple negative/basal-like type is ER-negative, PR-negative, and HER2-negative.

In addition, in the present invention, the biological sample includes blood and urine.

Furthermore, as another exemplary embodiment of the present invention, the ANT2 and VDAC1 genes are genes involved in tumor initiation and proliferation.

Further, as still another exemplary embodiment of the present invention, the HCCR1 gene is a gene involved in tumor progression.

In addition, as yet another exemplary embodiment of the present invention, the TGF-β3 gene is a gene involved in a tumor immune response.

Furthermore, as yet another exemplary embodiment of the present invention, the caveolin-1 and β-catenin genes are genes involved in cancer stem cells and anticancer agent resistance.

Further, the present invention provides a composition for early diagnosis of breast cancer using exosomes, the composition including a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

In addition, the present invention provides a kit for early diagnosis of breast cancer using exosomes, the kit including a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

As another exemplary embodiment of the present invention, the material which measures the mRNA levels may be a primer or probe capable of amplifying mRNA.

Furthermore, the present invention provides a method for monitoring recurrence after treatment of breast cancer, the method including: (a) extracting mRNA from exosomes isolated from biological samples of a normal person and a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2 and VDAC1 genes are increased compared to the normal person as breast cancer recurrence.

As an exemplary embodiment of the present invention, the subject may be a person who has received breast cancer treatment.

Further, the present invention provides a kit for monitoring recurrence after treatment of breast cancer, the kit including a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

### [Advantageous Effects]

The present inventors have developed a technique for analyzing mRNA in exosomes having a double lipid membrane structure (lipid bilayer) which can be protected from ribonuclease (RNase) in blood and determined that diagnostic accuracy and reproducibility can be increased through the technique. Since breast cancer is clearly classified into molecular subtypes and targeted therapy is effectively performed, the present invention enables four molecular subtypes of breast cancer to be early diagnosed through a minimal multiple cancer gene biomarker technique applicable to the early diagnosis of breast cancer according to the molecular subtypes thereof, can determine breast cancer progression stages according to the expression levels of the molecular subtypes, and makes it possible to monitor recurrence after treatment of breast cancer using the analysis of multiple gene expression in exosomes due to the technical feature of high detection sensitivity. In addition, although the regular examination items for monitoring the recurrence of breast cancer, which are currently being performed, the regular examination items have disadvantages of not only being expensive, but also causing inconvenience to an examinee and requiring a lot of time, since a product developed in the present invention is extremely useful in terms of the fact that results can be obtained with high detectability and low cost, simply and in a short time without pain to an examinee, the product is expected to be widely used for developing a medical instrument for ex-vivo gene diagnosis for monitoring recurrence after treatment of breast cancer.

### [Description of Drawings]

FIG. 1 is a graph showing the results of analyzing expression levels by treating breast cancer subtype cell lines with 6 biomarker genes (ANT2, VDAC1, HCCR1, caveolin-1, β-catenin, and TGF-β3) selected from breast cancer-derived exosomes.
FIG. 2 is a graph confirming the expression levels of pooling samples of healthy people and patients with breast cancer using 5 biomarker genes (ANT2, VDAC1, HCCR1, caveolin-1, and TGF-β3) obtained from breast cancer-derived exosomes.
FIG. 3 is a graph confirming the Ct values of real-time PCR when exosomes secreted by 5x10⁵ or more breast cancer cells (MDA-MB-231) for 48 hours were spiked.

### [Modes of the Invention]

The present inventors have developed a technique for analyzing mRNA in exosomes having a double lipid membrane structure (lipids layer) which can be protected from a ribonuclease (RNase) in blood and determined that diagnostic accuracy and reproducibility can be increased through the technique, and thus confirmed based on this that there is a possibility of developing a method for early diagnosis of breast cancer by using liquid biopsy multiple cancer gene biomarkers and a medical instrument for ex-vivo gene diagnosis for monitoring recurrence after treatment of breast cancer, thereby completing the present invention.

Further, since breast cancer is clearly classified into molecular subtypes and targeted therapy is effectively performed, the present inventors have found a minimal multiple cancer gene biomarker applicable to the early diagnosis of breast cancer according to the molecular subtypes thereof, and intend to provide a method for early diagnosis of breast cancer according to four molecular subtypes thereof, a method for determination of breast cancer progression stages according to the expression levels of the molecular subtypes, monitoring capable of confirming recurrence after treatment of breast cancer due to the technical feature of high detection sensitivity, a method capable of classifying cancer patients into a high-risk group and a low-risk group based on a specific gene biomarker expression level, and the like.

Hereinafter, the present invention will be described in detail.

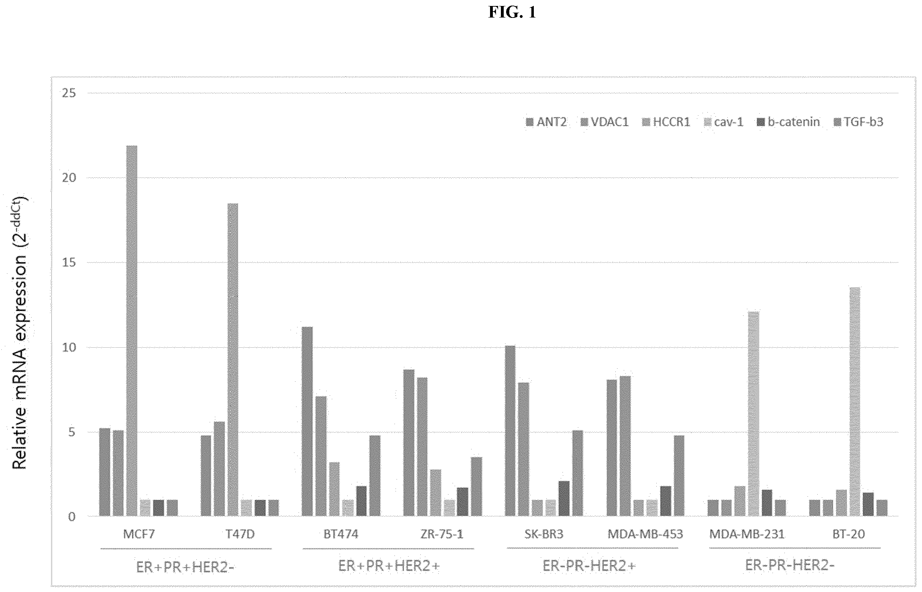

In an exemplary embodiment of the present invention, 6 genes (ANT2, VDAC1, HCCR1, caveolin-1, β-catenin, and TGF-β3) were selected by experiments using exosomes derived from breast cancer cell lines as illustrated in FIG. 1, the expression levels thereof were independently analyzed three or more times for each of two breast cancer molecular subtype cell lines (MCF7, T47D / BT474, ZR-75-1 / SK-BR3, MDA-MB453 / MDA-MB-231, BT20). As a result, ANT2 and VDAC1, which play an important role in tumor initiation and proliferation, exhibited a positive correlation with ER- and PR-positive and HER2-positive, respectively, and a HCCR1 gene involved in tumor progression exhibited a positive correlation with ER-positive and PR-positive. Further, caveolin-1 and β-catenin, which play an important role in cancer stem cells and anticancer agent resistance, were expected to have a deep correlation with a triple negative molecular subtype (ER-PR-HER2-), but contrary to the expectation, only caveolin-1 was observed to have, specifically, high expression in the triple negative molecular subtype, and in the case of TGF-β3, a positive correlation with HER2-positive was confirmed (see Example 1).

In another exemplary embodiment of the present invention, as a result of confirming the expression level of five biomarker genes obtained from breast cancer-derived exosomes for pooling samples of healthy people and patients with breast cancer, it was confirmed that ANT2 and VDAC1 exhibited the largest difference regardless of breast cancer molecular subtype (see Example 2).

In still another exemplary embodiment of the present invention, in order to experimentally discover that the present invention has high sensitivity and prove that the present invention is useful for monitoring the recurrence of breast cancer, as a result of attempting to predict the number of detectable cancer cells by subjecting normal blood to spike-testing in which the normal blood was spiked with breast cancer cell lines (MDA-MB-231 : ER-/PR-/HER2-), it was confirmed that when exosomes secreted by 5x10⁵ breast cancer cells (MDA-MB-231) for 48 hours were spiked, the Ct value of real-time PCR was less than 35, and as a result, it was confirmed that the value was within a gene detectable range (see Example 3).

Therefore, the present invention provides a method for providing information for early diagnosis of breast cancer, the method including: (a) extracting mRNA from exosomes isolated from biological samples of a normal person and a subject; (b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and (c) determining a case where the mRNA levels of the ANT2 and VDAC 1 genes are increased compared to the normal person as breast cancer.

As used herein, the term "exosome" comprehensively refers to a small vesicle having a double lipid membrane (lipid bilayer) secreted from cells, and the exosome is known to be secreted from various cells and have a diameter of approximately 30 to 200 nm. These exosomes include various types of proteins, DNA, mRNA, miRNA, and the like derived from cells.

As used herein, the term "biological sample" refers to all samples including exosomes, capable of being non-invasively obtained, and is preferably blood, plasma, serum, bone marrow, a tissue, a cell, saliva, sputum, hair, urine and the like, and more preferably blood, urine and the like, but is not limited thereto as long as the biological sample is a sample where it is possible to measure the amount of mRNAs of one or more multiple cancer gene biomarkers selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 1 (TGF-β3) genes.

As used herein, the term "molecular subtype" refers to a classification type according to molecular diagnosis for clinical use such as diagnosis and treatment of a disease, and recently, while it has become possible to profile a patient's body at the molecular level, there is a tendency that the profiling technique leads to the development of a targeted therapeutic method, and even the classification method of the disease has changed. Molecular subtypes according to the classification are closely associated with cancer treatment and prognosis of cancer patients, and are preferably breast cancer, and the associated molecular subtypes of breast cancer are Luminal A, Luminal B, HER2 type, and Triple negative/basal-like, but are not limited thereto.

Further, among the molecular subtypes, the Luminal A type is characterized by being estrogen receptor-positive (ER-positive), progesterone receptor-positive (PR-positive), and human epidermal growth factor receptor 2-negative (HER2-negative), molecular subtype Luminal B is characterized by being ER-positive, PR-positive, and HER2-positive, the molecular subtype HER2 type is ER-negative, PR-negative, and HER2-positive, and the molecular subtype Triple negative/basal-like type is characterized by being ER-negative, PR-negative, and HER2-negative, but the molecular subtypes are not limited thereto.

The ANT2 and VDAC1 genes are characterized by being genes involved in tumor initiation and proliferation, the HCCR1 gene is characterized by being a gene involved in tumor progression, the TGF-β3 gene is characterized by being a gene involved in a tumor immune response, and the caveolin-1 and β-catenin genes are characterized by being genes involved in cancer stem cells and anticancer agent resistance, but the genes are not limited thereto.

Further, the present invention provides a composition for early diagnosis of breast cancer using exosomes, the composition including a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

In addition, the present invention provides a kit for early diagnosis of breast cancer using exosomes, the kit including a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

The exosomes may be isolated from blood or urine, but are not limited thereto, and the material which measures the mRNA level is characterized by being a primer or probe capable of amplifying mRNA, but is not limited thereto.

Hereinafter, preferred examples for helping the understanding of the present invention will be suggested. However, the following examples are provided only to more easily understand the present invention, and the contents of the present invention are not limited by the following examples.

### [Examples]

### Example 1. Selection of multiple gene biomarkers for early diagnosis of breast cancer

### 1-1. Culture of breast cancer cell lines for each molecular subtype

Breast cancer cell lines for each molecular subtype as shown in the following [Table 1] were purchased from the Korean Cell Line Bank and then cultured.

**[Table 1]**

| Molecular subtype | Breast cancer cell line |
|---|---|
| ER+/PR+/HER2- | MCF7, T47D (Luminal A) |
| ER+/PR+/HER2+ | BT474, ZR-75-1 (Luminal B) |
| ER-/PR-/HER2+ | SK-BR3, MDA-MB-453 (HER2 type) |
| ER-/PR-/HER2- | MDA-MB-231, BT20 (Triple negative/basal-like) |

As the culture conditions, the breast cancer cell lines were cultured in a cell incubator under the conditions of 5% CO₂ and 37°C using a Dulbecco's Modified Eagle's medium (DMEM) containing 10% fetal bovine serum (FBS) and 1% penicillin/streptomycin.

### 1-2. Extraction of breast cancer cell line-derived exosomes

In order to extract the exosomes secreted by the breast cancer cell line, a cell culture solution containing exosome-depleted FBS from which the exosomes had been removed was used. Before the culture solution was changed, the culture solution was washed twice with phosphate buffered saline (PBS, pH 7.4), and after the culture solution was changed, the enrichment of exosomes was induced by culturing the breast cancer cell lines in a cell incubator under the conditions of 5% CO₂ and 37°C for 48 hours, and after the volume of the culture solution was concentrated to 1/10 using an Amicon Ultra-15 centrifugal filter (Ultracel-3K), exosomes were obtained using an SBI Exoquick TC reagent.

### 1-3. Extraction of RNAs in exosomes

RNAs in exosomes were extracted using an RNA extraction reagent for exosomes produced and sold by Genolution, Inc. In this case, as the principle of extracting RNAs in exosomes, exosomes in the culture solution were lysed and only RNAs were isolated from the proteins, DNA, and RNA (mRNA, miRNA, rRNA, and the like) that flowed out of the exosomes, and in this case, an extraction process (protocol) was constructed, which can increase the purity and maximize the RNA extraction concentration (RNA prep yield) by optimizing the proteins and DNA such that they were not contaminated.

### 1-4. Quantification of RNA in extracted exosomes

In order to quantify the concentration of RNA in exosomes extracted from the breast cancer cell line culture solution, the concentration was measured using NanoDrop (absorbance at a wavelength of 260 nm), RNA purity and contamination were confirmed by a 260/280 ratio and a 260/230 ratio, and RNA belonging to the normal range was used in the experiment.

### 1-5. Quantitative reverse transcription-polymerase chain reaction (qRT-PCR)

The One-step qRT-PCR reagent (SensiFAST™ Probe Lo-ROX One-Step Kit), which is a product of Bioline, was used, in which a reverse transcription and a cDNA amplification process (polymerase chain reaction) are performed as a one-step using 100 ng of breast cancer cell line-derived exosome RNA as a template, and each gene probe is a product of Integrated DNA Technologies (IDT), and the information on the product is shown in [Table 2] below.

**[Table 2]**

| Gene name | | Cat No. (assay name) from IDT Company |
|---|---|---|
| **Target gene (Probe cat No. from IDT company)** | | |
| ① | ANT2 | Hs.PT.56a.39886014.g |
| ② | VDAC1 | Hs.PT.58.2339327.g |
| ③ | HCCR-1 | Hs.PT.58..1837203 |
| ④ | Caveolin-1 | Hs.PT.56a.40058555.g |
| ⑤ | β-catenin | Hs.PT.58.3699397 |
| ⑥ | TGF-β3 | Hs.PT.58.27186053.g |

| **Internal control gene (RNA standardization)** | | |
|---|---|---|
| ⑦ | b-actin | Hs.PT.56a.40703009.g |
| ⑧ | 18S rRNA | Hs.PT.39a.22214856.g |

The real-time PCR instrument used in the experiment was a StepOne plus instrument of ABI systems, and all samples were duplicated to increase the reliability of the results.

### 1-6. Selection and analysis results

As a breast cancer early diagnosis biomarker candidate, 10 or more candidate genes were selected from among genes involved in tumor initiation and proliferation, genes involved in tumor progression, genes involved in tumor immune responses, and genes involved in cancer stem cells and anticancer agent resistance, and preliminary experiments were performed on cancer cell line-derived exosomes, genes with a real-time qRT-PCRCt value of 35 or higher based on 100 ng of RNAs concentration were excluded, and 6 genes showing expression above a certain level in cancer cell line-derived exosomes were selected.

Among the 6 genes, ANT2 and VDAC1 genes are involved in tumor initiation and proliferation, a HCCR1 gene is involved in tumor progression, TGF-β3 is involved in a tumor immune response, and caveolin-1 and β-catenin genes are involved in cancer stem cells and anticancer agent resistance.

The expression levels of the selected 6 genes were independently analyzed three or more times or each of two breast cancer molecular subtype cell lines (MCF7, T47D / BT474, ZR-75-1 / SK-BR3, MDA-MB453 / MDA-MB-231, BT20). In this case, two genes (b-actin and 18S rRNA) were used as an internal control (housekeeping gene) gene for normalizing RNA concentration, and the relative expression levels were compared by setting the expression of a subtype with the lowest expression of each gene to 1.

As a result of the analysis, by obtaining a result of repeatedly showing a meaningful difference in a specific molecular subtype, 5 genes out of 6 target genes are expected to function as a very useful biomarker for early diagnosis of each breast cancer molecular subtype.

Specifically, as illustrated in FIG. 1, ANT2 and VDAC1 genes, which play an important role in tumor initiation and proliferation, exhibited a positive correlation with ER- and PR-positive and HER2-positive, respectively, and a HCCR1 gene involved in tumor progression exhibited a positive correlation with ER-positive and PR-positive. Further, caveolin-1 and β-catenin, which play an important role in cancer stem cells and anticancer agent resistance, were expected to have a deep correlation with a triple negative molecular subtype (ER-PR-HER2-), but contrary to the expectation, only caveolin-1 was observed to have, specifically, high expression in the triple negative molecular subtype, and TGF-β3 exhibited a positive correlation with HER2-positive.

When taken together, the breast cancer molecular subtype Luminal A type (ER+/PR+/HER2-) exhibited a positive correlation with ANT2, VDAC1, and HCCR1 because only ER and PR are positive, and accordingly, it could be confirmed that the three genes are Luminal A type-associated gene markers. In addition, in the case of a molecular subtype Luminal B type (ER+/PR+/HER2+) of breast cancer, since ER and PR are positive and HER2 is also positive, in the case of HER2-negative, the HCCR1 gene is excluded because the HCCR1 gene does not show a positive correlation, and instead, TGF-β3 shows a positive correlation with HER2-positive, so that it could be confirmed that ANT2, VDAC1, and TGF-β3 genes having a positive correlation with ER-positive, PR-positive, and HER2-positive were Luminal B type-associated gene markers.

Furthermore, in the case of the breast cancer molecular subtype HER2 type (ER-/PR-/HER2+), since only HER2 is positive, it can be confirmed that ANT2, VDAC1, and TGF-β3 genes having a positive correlation with HER2 are also HER2 type-associated gene markers, and finally, in the case of the breast cancer molecular subtype Triple negative/basal-like type, since all of ER, PR, and are negative, only caveolin-1 is specifically expressed at a high level, so that it could be confirmed that the caveolin-1 gene is a Triple negative/basal-like type gene marker.

Finally, the five gene markers that show the most obvious differences for each molecular subtype are summarized in the following [Table 3].

**[Table 3]**

| **Molecular subtype** | **Gene** | | | |
|---|---|---|---|---|
| ER+/PR+/HER2- | ANT2, VDAC1 | HCCR1 | | |
| ER+/PR+/HER2+ | ANT2, VDAC1 | | | TGF-β3 |
| ER-/PR-/HER2+ | ANT2, VDAC1 | | | TGF-β3 |
| ER-/PR-/HER2- | | | Cav-1 | |

The five gene combinations are expected to not only enable early diagnosis of breast cancer for each molecular subtype, but also be very usefully used for monitoring recurrence after treatment of breast cancer due to the technical feature of high detection sensitivity. In addition, the expression level of gene biomarkers specifically expressed in the triple negative molecular subtype with the worst treatment prognosis is expected to indirectly predict the prognosis of breast cancer treatment.

### Example 2. Comparison of multiple gene biomarkers for early diagnosis of normal person and breast cancer

### 2-1. Preparation of blood samples for healthy people and patients with cancer

The blood samples of healthy people and patients with cancer used in this study were samples stored frozen (-80°C), and serum collected in SST tubes was used. Specifically, in the case of healthy people, samples within a normal range of biomarkers for cancer protein diagnosis were used, and in the case of patients with cancer, samples that were diagnosed as cancer (confirmed) at a university hospital were used. The minimum volume of serum required to extract RNA in exosomes was 200 ul, and as the sample, a breast cancer patient pooling sample was prepared as follows.

**[Table 4]**

| | | |
|---|---|---|
| **Healthy people serum** | Male | Prepare 1 ml of samples for 5 people by combining 200 ul of each sample |
| | Female | Prepare 1 ml of samples for 5 people by combining 200 ul of each sample |
| **Breast cancer serum** | stage 1 & 2 | Prepare 1 ml of samples for 5 people by combining 200 ul of each sample |
| | stage 3 & 4 | Prepare 1 ml of samples for 5 people by combining 200 ul of each sample |

In principle, the samples prepared as described above were aliquoted into 200 ul, respectively and stored at -80°C, and all the samples were subjected to freezing and thawing one time.

### 2-2. Extraction of RNAs in exosomes

Nextractor NX-48, an automated device produced and sold by Genolution, Inc., was used to extract RNA in exosomes from sera of healthy people and patients with breast cancer, and as a reagent, a reagent for extracting RNA in exosomes from the same company, which is most suitable for the device, was used. The principle of extracting RNA in exosomes is as described in Example 1-3.

### 2-3. Quantification of RNA in extracted exosomes

The method for quantifying the concentration of RNA in exosomes extracted from serum by an automated device is as described in Example 1-4.

### 2-4. Quantitative reverse transcription-polymerase chain reaction (qRT-PCR)

Using 100 ng of exo RNA extracted from serum as a template, qRT-PCR was performed in the same manner as in Example 1-5.

### 2-5. Comparative results

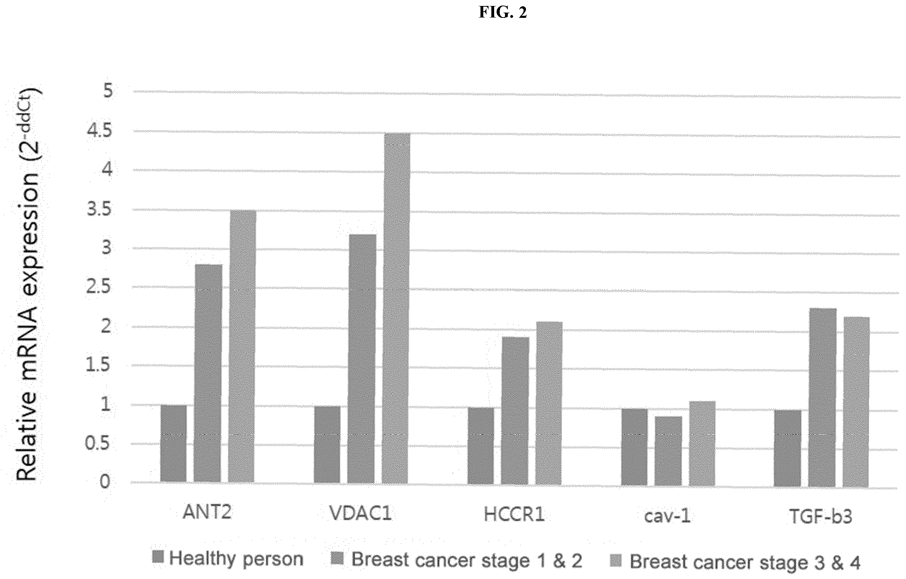

As illustrated in FIG. 2, as a result of confirming the expression levels of the five biomarker genes obtained from breast cancer-derived exosomes for pooling samples of healthy people and patients with breast cancer, a significant difference was shown between healthy people and patients with breast cancer.

In this case, caveolin-1 corresponding to triple negative, which was not a pooling sample for each molecular subtype, showed a relatively low difference between normal people and patients with breast cancer. Accordingly, the difference needs to be confirmed again in the sample for each molecular subtype, and it is determined that ANT2 and VDAC1 which show the largest difference regardless of the molecular subtype of breast cancer are useful for early diagnosis of breast cancer.

### Example 3. Confirmation of possibility of monitoring recurrence after treatment of breast cancer through multiple gene biomarkers

To experimentally prove the detection sensitivity, which is the scientific basis for this technique, is useful for breast cancer recurrence monitoring, it was attempted to predict the number of detectable cancer cells by subjecting normal blood to spike testing in which the normal blood was spiked with breast cancer cell lines (MDA-MB-231: ER-/PR-/HER2-).

Assuming that the blood of a woman weighing 60 kg is 3.6 L, it was assumed that when there was tumor having a size of 1 cm³ (number of cancer cells: 1x10⁹), exosomes secreted by 2.22x10⁶ cancer cells were present in 1 ml of blood.

**[Table 5]**

| Female | |
|---|---|
| Body weight | 60kg |
| blood | 3.6L (3,600ml) |
| Tumor size | 1cm³ (1x10⁹) |
| Blood 1ml | 2.22x10⁶ tumor cells-derived exosomes |

Generally, when 1 ml of blood is centrifuged, about 0.5 ml of serum is obtained, so that when 0.25 ml of serum from a healthy person was subjected to spike testing with exosomes secreted by 1x10⁵ to 1x10⁷ breast cancer cells for 48 hours under the artificial conditions in which tumors with a size of 1 cm³ size were present, it was tried to confirm a range where gene analysis was possible.

**[Table 6]**

| | Blood 1ml (serum 0.5ml) |
|---|---|
| Tumor 1cm³ | 2x10⁶ |
| Normal serum 0.25ml | 1x10⁶ |

Since the breast cancer cell line-derived exosomes used in the spike test are exosomes secreted by MDA-MB-231 (ER-PR-HER2-) cells, a gene to be analyzed was analyzed using caveolin-1.

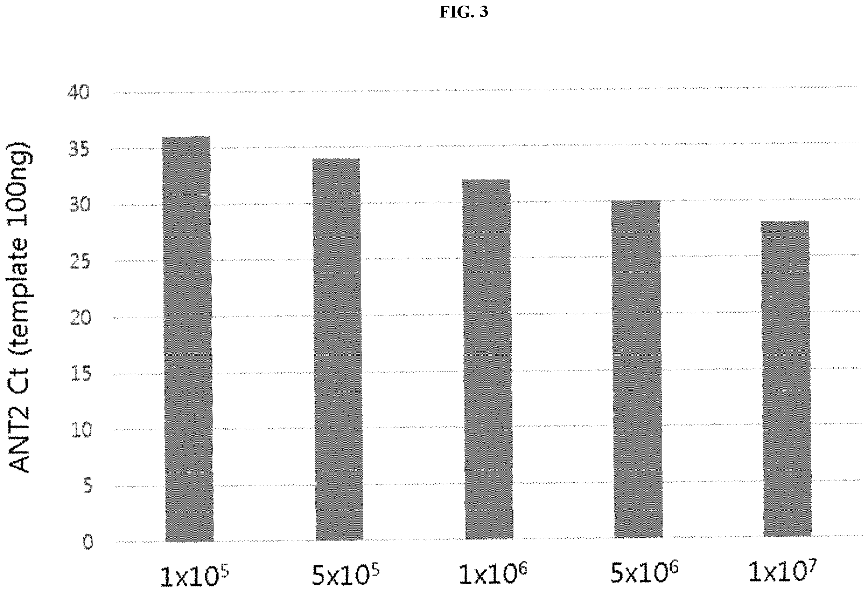

As a result, as illustrated in FIG. 3, the Ct value of real-time PCR was less than 35 when the exosomes secreted by 5x10⁵ or more breast cancer cells (MDA-MB-231) for 48 hours were spiked, so that it was determined to be within a gene-detectable range (generally, when the Ct value of real-time PCR is 35 or more, the gene expression value is not trusted). When a person actually has tumors, the exosomes in blood are accumulated for longer than 48 hours, so that actually, this technique is a highly sensitive detection technique capable of distinguishing examinees who have tumors smaller than 1 cm³ in size, and is predicted to be not only utilized simply for early diagnosis of breast cancer, but also very useful for monitoring recurrence after treatment of breast cancer.

Although specific parts of the present invention have been described in detail, it will be obvious to those skilled in the art that such a specific description is just a preferred embodiment and the scope of the present invention is not limited thereby. Accordingly, the substantial scope of the present invention will be defined by the appended claims and equivalents thereof.

### [Industrial Applicability]

When the minimal multiple cancer gene biomarker technology applicable to the early diagnosis of breast cancer according to the molecular subtypes of the present invention is used, it is possible to early diagnose breast cancer according to four molecular subtypes thereof, it is possible to determine the degree of breast cancer progression depending on the degree of expression, and it is possible to monitor recurrence after treatment of breast cancer using the analysis of multiple gene expression in exosomes due to the technical feature of high detection sensitivity. In addition, the regular examination items for monitoring the recurrence of breast cancer, which are currently being performed, have disadvantages of not only being expensive, but also causing inconvenience to an examinee and requiring a lot of time, but since a product developed in the present invention is extremely useful in terms of the fact that results can be obtained with high detectability and low cost, simply and in a short time without pain to an examinee, the product is expected to be widely used for developing a medical instrument for ex-vivo gene diagnosis for monitoring recurrence after treatment of breast cancer.

## Claims

1. A method for providing information for early diagnosis of breast cancer, the method comprising:
(a) extracting mRNA from exosomes isolated from biological samples of a normal person and a subject;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2 and VDAC1 genes are increased compared to the normal person as breast cancer.

2. The method of claim 1, wherein the breast cancer is selected from the group consisting of Luminal A, Luminal B, HER2 type, and Triple negative/basal-like molecular subtypes.

3. A method for providing information for early diagnosis of molecular subtype Luminal Abreast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and HCCR1 genes are increased compared to the mRNA levels of caveolin-1, β-catenin, and TGF-β3 genes as molecular subtype Luminal A breast cancer.

4. The method of claim 3, wherein the molecular subtype Luminal A type is estrogen receptor-positive (ER-positive), progesterone receptor-positive (PR-positive), and human epidermal growth factor receptor 2-negative (HER2-negative).

5. A method for providing information for early diagnosis of molecular subtype Luminal B breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and TGF-β3 genes are increased compared to the mRNA levels of caveolin-1, and β-catenin genes as molecular subtype Luminal B breast cancer.

6. The method of claim 5, wherein the molecular subtype Luminal B type is ER-positive, PR-positive, and HER2-positive.

7. A method for providing information for early diagnosis of molecular subtype HER2 type breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and TGF-β3 genes are increased compared to the mRNA levels of HCCR1, caveolin-1, and β-catenin genes as molecular subtype HER2 type breast cancer.

8. The method of claim 7, wherein the molecular subtype HER2 type is ER-negative, PR-negative, and HER2-positive.

9. A method for providing information for early diagnosis of molecular subtype Triple negative/basal-like breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA level of the caveolin-1 gene is increased compared to the mRNA levels of ANT2, VDAC1, HCCR1, β-catenin, and TGF-β3 genes as molecular subtype Triple negative/basal-like breast cancer.

10. The method of claim 9, wherein the molecular subtype Triple negative/basal-like type is ER-negative, PR-negative, and HER2-negative.

11. The method of any one of claims 3, 5, 7, and 9, wherein the subject is a patient with breast cancer.

12. The method of any one of claims 1, 3, 5, 7, and 9, wherein the biological sample is blood or urine.

13. The method of any one of claims 1, 3, 5, 7, and 9, wherein the ANT2 and VDAC1 genes are genes involved in tumor initiation and proliferation.

14. The method of any one of claims 1, 3, 5, 7, and 9, wherein the HCCR1 gene is a gene involved in tumor progression.

15. The method of any one of claims 1, 3, 5, 7, and 9, wherein the TGF-β3 gene is a gene involved in a tumor immune response.

16. The method of any one of claims 1, 3, 5, 7, and 9, wherein the caveolin-1 and β-catenin genes are genes involved in cancer stem cells and anticancer agent resistance.

17. A composition for early diagnosis of breast cancer using exosomes, the composition comprising a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

18. The composition of claim 17, wherein the exosomes are isolated from blood or urine.

19. The composition of claim 17, wherein the material which measures the mRNA levels is a primer or probe capable of amplifying mRNA.

20. A kit for early diagnosis of breast cancer using exosomes, the kit comprising a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

21. The kit of claim 20, wherein the exosomes are isolated from blood or urine.

22. The kit of claim 20, wherein the material which measures the mRNA levels is a primer or probe capable of amplifying mRNA.

23. A method for monitoring recurrence after treatment of breast cancer, the method comprising:
(a) extracting mRNA from exosomes isolated from biological samples of a normal person and a subject;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2 and VDAC1 genes are increased compared to the normal person as breast cancer recurrence.

24. A kit for monitoring recurrence after treatment of breast cancer, the kit comprising a material which measures the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes.

25. A method for early diagnosis of breast cancer, the method comprising:
(a) extracting mRNA from exosomes isolated from biological samples of a normal person and a subject;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2 and VDAC1 genes are increased compared to the normal person as breast cancer.

26. A method for early diagnosis of molecular subtype Luminal A breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and HCCR1 genes are increased compared to the mRNA levels of caveolin-1, β-catenin, and TGF-β3 genes as molecular subtype Luminal A breast cancer.

27. A method for early diagnosis of molecular subtype Luminal B breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and TGF-β3 genes are increased compared to the mRNA levels of caveolin-1, and β-catenin genes as molecular subtype Luminal B breast cancer.

28. A method for early diagnosis of molecular subtype HER2 type breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA levels of the ANT2, VDAC1, and TGF-β3 genes are increased compared to the mRNA levels of HCCR1, caveolin-1, and β-catenin genes as molecular subtype HER2 type breast cancer.

29. A method for early diagnosis of molecular subtype Triple negative/basal-like breast cancer, the method comprising:
(a) extracting mRNA from exosomes isolated from a biological sample of a subj ect;
(b) measuring the levels of one or more mRNAs selected from the group consisting of adenine nucleotide translocase 2 (ANT2), voltage-dependent anion-selective channel 1 (VDAC1), human cervical cancer oncogene (HCCR1), caveolin-1, β-catenin, and transforming growth factor-beta 3 (TGF-β3) genes using the extracted mRNA as a template; and
(c) determining a case where the mRNA level of the caveolin-1 gene is increased compared to the mRNA levels of ANT2, VDAC1, HCCR1, β-catenin, and TGF-β3 genes as molecular subtype Triple negative/basal-like breast cancer.
